# EUROPEAN PATENT SPECIFICATION

(11) **EP 1 698 281 B1**
(45) Date of publication and mention of the grant of the patent: **29.07.2009**
(21) Application number: 06004247.0
(22) Date of filing: 02.03.2006
(51) Int. Cl.: A61B 8/12, A61M 25/10

(54) **Ultrasonic probe**
Ultraschallsonde
Sonde à ultrasons

(30) Priority: 04.03.2005 JP 2005061156
(43) Date of publication of application: 06.09.2006
(73) Proprietor: FUJIFILM Corporation, Minato-ku Tokyo (JP); Fujinon Corporation, Saitama-shi, Saitama (JP)
(72) Inventor: Osawa, Atsushi c/o Fuji Photo Film Co., Ltd., Ashigarakami-gun Kanagawa (JP); Tanaka, Toshizumi c/o Fujinon Corporation, Saitama-shi Saitama (JP); Itoi, Hiromu c/o Fujinon Corporation, Saitama-shi Saitama (JP)
(74) Representative: Klunker, Hans-Friedrich

(56) References cited:
- EP-A- 0 466 424
- EP-A- 0 940 123
- US-A- 5 400 785
- US-A- 5 846 205
- US-B1- 6 689 066

## Description

### BACKGROUND OF THE INVENTION

### 1. Field of the Invention

The present invention relates to an ultrasonic probe according to the preamble of claim 1. Such a probe scans inside a living organism by ultrasound with the use of an ultrasonic transducer.

### 2. Description Related to the Prior Art

Recently, in a field of medical care, a medical diagnosis using ultrasonic images is widely used. The ultrasonic image is obtained by emitting ultrasonic waves to a required area of a living organism from an ultrasonic probe, and electrically detecting echo signal reflected from the living organism. In addition, when the ultrasonic waves scan the living organism, an ultrasonic tomography can be obtained. For this purpose, it is known an ultrasonic probe having a scanning mechanism for mechanically rotating, swinging or sliding an ultrasonic transducer which transmits/receives the ultrasonic waves. To obtain an ultrasonic image of inside the living organism with use of such ultrasonic probes, an operator inserts the ultrasonic probe into a forceps inlet of an endoscope, and follows the position of a leading end of the ultrasonic probe in the living organism by use of a camera provided at leading edge of the endoscope.

The ultrasonic transducer includes a piezoelectric element, electrodes and an acoustic matching layer, and is contained in a cap for sealing the leading end of the ultrasonic probe. Particularly, in the ultrasonic probe with the scanning mechanism, the cap is filled with suitable liquid so as to improve a transmission efficiency of the ultrasonic waves and to smooth the movement of the ultrasonic transducer (for example Japanese Patent Laid-Open Publication No. 2002-345819). The cap is also essential for protecting the moving ultrasonic transducer.

In general, the cap is formed of a resin material, such as polyethylene. However, the resin has high acoustic impedance which cannot match that of the living organism, which lowers receiver sensitivity of the ultrasonic transducer. One solution for matching the acoustic impedance between the cap and the living organism is to change the material used for the cap. However, to use a new resin material as an alteration of known current resin material, an enormous cost and work is needed to research and test the new material so as to confirm biocompatibility thereof. Accordingly, the use of new material is not practical.

In accordance with the preamble of claim 1, EP 0 940 123 discloses an ultrasonic probe in which the cap is formed by a wire coil. By spreading apart windings of the coil, a window region is formed.

US-A-5,400,785 discloses an ultrasonic probe having a cap, which includes a plurality of openings which are covered by a material which prevents passage of liquids through the openings.

### SUMMARY OF THE INVENTION

An object of the present invention is to provide an ultrasonic probe which has improved receiver sensitivity with using a general material for a cap.

In order to achieve the above object and other objects, an ultrasonic probe of the present invention comprises the features of claim 1.

It is preferable that the low material density part is an opening. In addition, it is preferable that liquid is supplied into the cap and then flows outside the cap through the openings.

It is preferable that the ultrasonic probe further comprises a balloon attached to the cap for covering the openings, and the liquid flows into the balloon through inside the cap and the openings.

It is preferable that the high- and low-density area is formed by winding a wire around a frame.

It is also preferable that the low material density part is a concave.

It is preferable that the cap has a cylindrical shape and the density interval is gradually changed in an axial direction and/or a circumference direction of the cap. In addition, it is preferable that the density interval is gradually changed from the center toward the ends in the axial direction of the cap such that the acoustic impedance gradually becomes higher from the center toward the ends, so as to give an acoustic lens effect to the cap in the axial direction.

Since the low material density part is an opening, the balloon is attached to the cap for covering the openings, and the liquid flows into the balloon through inside the cap and the openings, a conventional dedicated liquid supply line to the balloon is not needed. Accordingly, the ultrasonic probe can be downsized.

### BRIEF DESCRIPTION OF THE DRAWINGS

The above objects and advantages of the present invention will become easily understood by one of ordinary skill in the art when the following detailed description would be read in connection with the accompanying drawings.
The embodiments shown in Fig. 2-8 do not fall under the scope of claim 1.
FIG.1 is a schematic view of an ultrasonic diagnosing system;
FIG.2 is a cross-sectional view of a leading end of an ultrasonic probe;
FIG.3 is an explanatory view of an electric structure of an ultrasonic transducer and a translator;
FIG.4 is a side view of a cap;
FIG. 5 is a graph showing an increase amount of receiver sensitivity in relation to an acoustic impedance of a meshed area;
FIG.6 is an explanatory view showing a forming method of the meshed area;
FIG.7 is a cross-sectional view of a leading end of an ultrasonic probe with a balloon attached around the cap;
FIG.8 is a cross-sectional view of a leading end of an ultrasonic probe with concaves in a cap;
FIG.9 is a cross-sectional view of a leading end of an ultrasonic probe in which an interval between holes in a cap gradually changes in an axial direction of the cap; and
FIG.10 is a cross-sectional view of a leading end of an ultrasonic probe in which a width of each hole in a cap gradually changes in the axial direction of the cap.

### PREFERRED EMBODIMENTS OF THE INVENTION

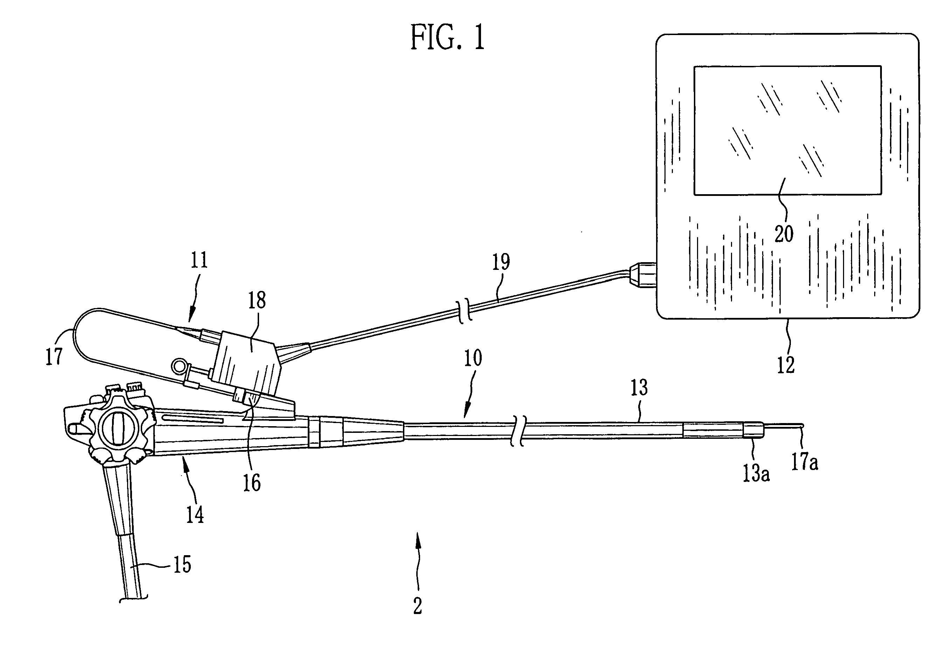

As shown in FIG.1, an ultrasonic diagnosing system 2 is composed of an endoscope 10, ultrasonic probe 11 and an ultrasonic observer 12. The endoscope 10 comprises a flexible insertion section 13 for being inserted into a living organism, an operation section 14 connected to a tail end of the insertion section 13, and a cord 15 connecting between the operation section 14 and a processor for the endoscope (not shown). At a leading end 13a of the insertion section 13, a camera (not shown) for capturing images of inside the living organism is incorporated. The image captured by the camera is displayed on a monitor for the endoscope (not shown) through the processor for the endoscope.

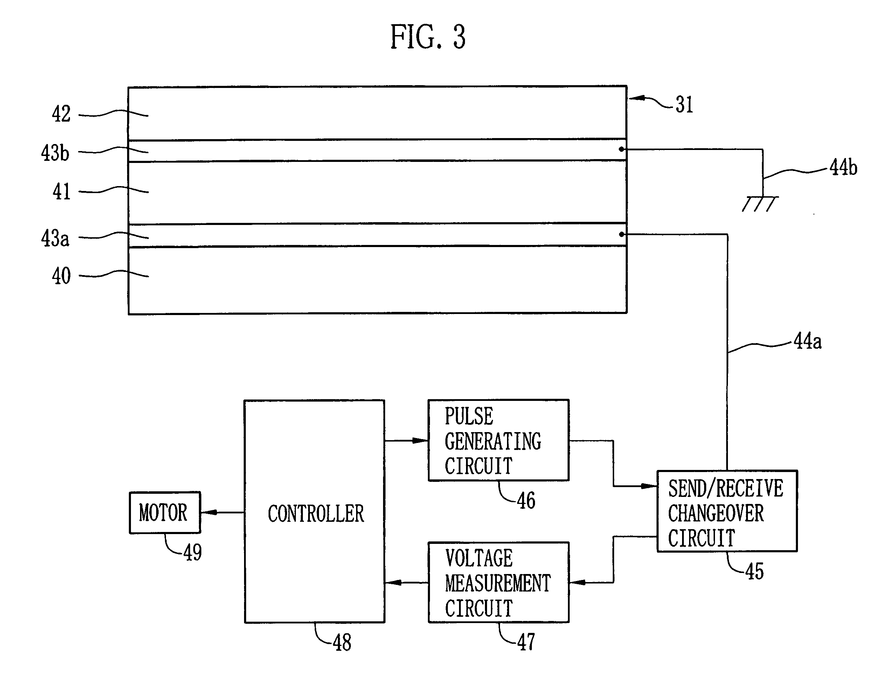

The ultrasonic probe 11 is composed of a flexible sheath 17 for penetrating the insertion section 13 from a forceps inlet 16 of the endoscope 10, a translator 18 incorporating a motor 49 and other members (refer to FIG.3) described later, and a cord 19 connecting between the translator 18 and the ultrasonic observer 12. The ultrasonic observer 12 has a monitor 20 for displaying an ultrasonic image.

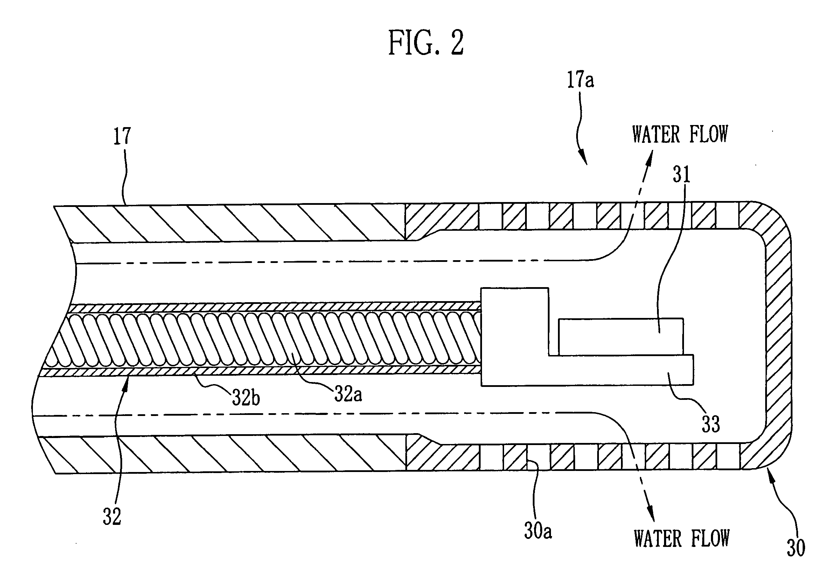

As shown in FIG.2, at a leading end 17a of the sheath 17, a cylindrical cap 30 formed of a resin material such as polyethylene is attached. In the cap 30, plural holes (openings) 30a are provided in an area where the ultrasonic waves pass through. The cap 30 covers an ultrasonic transducer 31, and a water inlet (not shown) is provided at a tail end of the sheath 17. When water is supplied into the sheath 17 from the water inlet, the water enters in the cap 30, and then flows out of the cap 30 through the holes 30a. By this water flow, the inside of the living organism can be filled with water.

The ultrasonic transducer 31 is attached on a base 33 to which a control cable 32 is connected. The control cable 32 is composed of a flexible shaft 32a and a flexible tube 32b for covering the flexible shaft 32a. A leading end of the flexible shaft 32a is connected to the base 33, and a tail end of the shaft 32a is inside the translator 18. The flexible shaft 32a is rotated at a predetermined revolution speed (for example 10 to 40 rolls per second) by the motor 49 (refer to FIG.3). Accordingly, the ultrasonic transducer 31 is rotated at the predetermined revolution speed around the flexible shaft 32a as a rotational axis. Note that since the water inside the cap 30 always flows, generation of air bubbles (cavitation) by the rotation of the ultrasonic transducer 31 is prevented.

As shown in FIG.3, the ultrasonic transducer 31 comprises a backing plate 40 formed of ferrite rubber, a piezoelectric element 41 formed of thin film of PZT (lead zirconate titanate) and an acoustic matching layer 42 formed of epoxy resin, which are laminated on the base 33 in this order. The piezoelectric element 41 is sandwiched between electrodes 43a and 43b. To the respective electrodes 43a and 43b, wires 44a and 44b are connected. The other side of the wire 44b for the electrode 43b is connected to earth. The other side of the wire 44a for the electrode 43a is connected to a send/receive changeover circuit 45 in the translator 18 through the control cable 32.

The send/receive changeover circuit 45 alternatively changes the ultrasonic transducer 31 to send or receive the ultrasonic waves at predetermined intervals. To the send/receive changeover circuit 45, a pulse generating circuit 46 and a voltage measurement circuit 47 is connected. The pulse generating circuit 46 applies a pulse voltage to the piezoelectric element 41 when the ultrasonic transducer 31 is going to generate the ultrasonic waves (the sending of ultrasonic wave). Thereby, the ultrasonic transducer 31 will generate the ultrasonic waves having a specific frequency.

The voltage measurement circuit 47 measures a voltage generated in the piezoelectric element 41 when the ultrasonic transducer 31 receives an echo signal from the living organism (the receiving of ultrasonic wave). The voltage measurement circuit 47 sends a measuring result to a controller 48. The controller 48 converts the received measuring result into an ultrasonic image, and sends this ultrasonic image to the ultrasonic observer 12.

To obtain the ultrasonic image of the inside of the living organism, at first the ultrasonic probe 11 is inserted from the forceps inlet 16 to penetrate the insertion section 13 of the endoscope 10. Then the insertion section 13 is inserted in the living organism. An operator searches a required area of the living organism while observing the monitor for the endoscope. When the leading end 17a of the sheath 17 reaches at the required area, the water is supplied inside the sheath 17, flows out of the cap 30 through the holes 30a, and fills the required area.

When an operation for obtaining the ultrasonic image is performed in this state, the ultrasonic transducer 31 rotates at the predetermined revolution speed. At the same time, the ultrasonic transducer 31 generates the ultrasonic waves by the application of the pulse voltage from the pulse generating circuit 46, while the send/receive changeover circuit 45 alternatively changes the ultrasonic transducer 31 to send or receive the ultrasonic waves at predetermined intervals. The living organism is scanned with the ultrasonic waves passing through the cap 30. When the echo signal from the living organism is received by the ultrasonic transducer 31, the voltage measurement circuit 47 measures the voltage generated in the piezoelectric element 41.

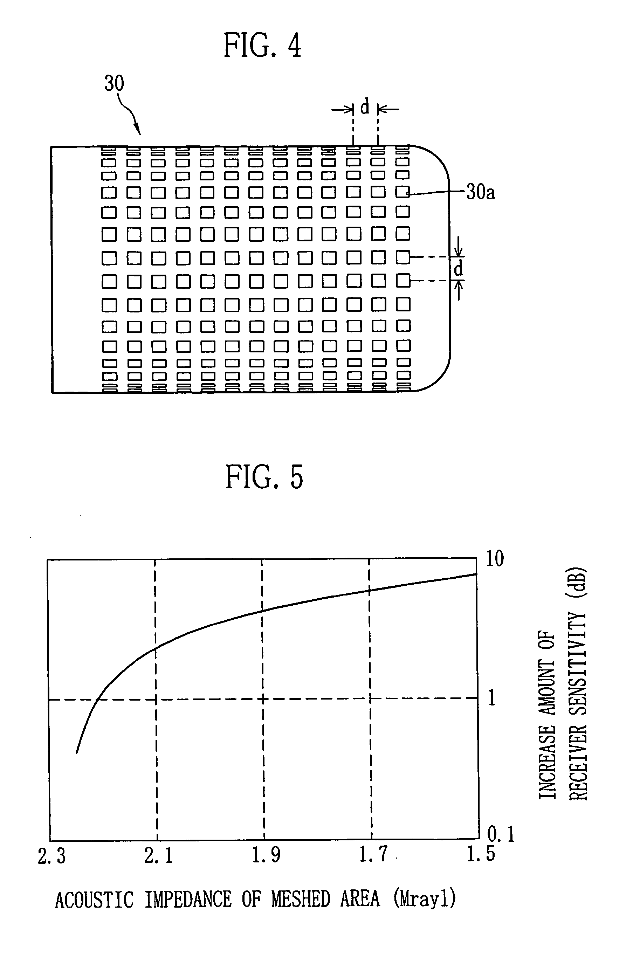

As shown in FIG. 4, the rectangular holes 30a are arranged in the cap 30 at regular intervals. A meshed area where the holes 30a are arranged is a high- and low-density area where low material density parts (holes 30a) and high material density parts (between the adjacent holes 30a) are alternately formed. The ultrasonic waves from/to the ultrasonic transducer 31 pass through this high- and low-density area. The density interval d (pitch of the holes 30a) is sufficiently smaller than a shortest wavelength λ of the ultrasonic waves, preferably below 5% of the wavelength λ (d<λ×0.05), more preferably below 1% of the wavelength λ (d<λ×0.01).

As a concrete example of the ultrasonic waves, when a center frequency thereof is 7.5MHz, and a band thereof is 90% of the center frequency, a bandwidth is 4 to 11MHz. Since a sonic velocity v in water is approximately 1500m/s, the shortest wavelength λ of the ultrasonic waves is calculated to (1500m/s) ÷ (11MHz)≈ 136µm. In this case, the density interval d is preferably below 6.8µm, more preferably below 1.4µm. Note that a length of one side of the hole 30a is approximately equal to the pitch of the holes.

When the density interval d is sufficiently smaller than the wavelength λ of the ultrasonic waves as describe above, the high- and low-density area is deemed uniform to the ultrasonic wave because an amplitude, a phase and a velocity of the ultrasonic wave are not affected. In addition, the acoustic impedance of the cap 30 is reduced from when there is no hole 30a (there is no high- and low-density area). Accordingly, a consistency of the acoustic impedances between the cap 30 and the living organism is improved, an ultrasonic reflectance of the cap 30 is lowered, and a receiver sensitivity of the ultrasonic transducer 31 is increased.

The acoustic impedance of the cap 30 can be reduced by reducing the density of the meshed area (a proportion of the high density parts). For example, when the cap 30 is formed of a polyethylene having approximately 2.3Mrayl of the acoustic impedance, the water having approximately 1.5Mrayl of the acoustic impedance fills the holes 30a (low density parts) and the density of the meshed area is 20 to 30%, the acoustic impedance of the meshed area of the cap 30 becomes approximately 1.7Mrayl. In this example, as shown in a graph of FIG.5, the receiver sensitivity is increased approximately 6 to 7dB from when the density of the meshed area is 100% (there is no hole 30a).

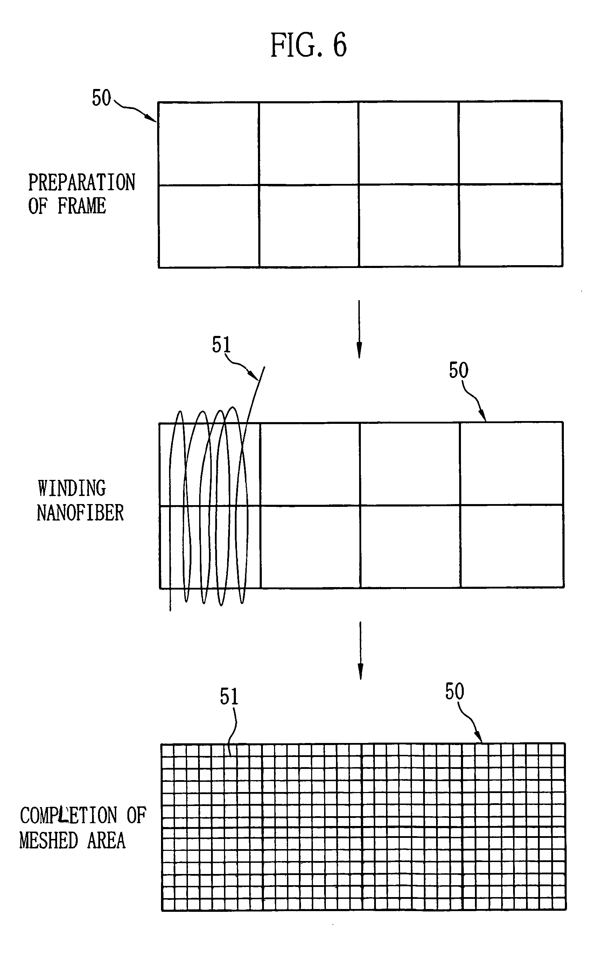

As shown in FIG.6, the meshed area of the cap 30 can be formed by winding a wire 51 around a lattice-shaped frame 50. The frame 50 is formed of a resin material such as polyethylene having approximately 100µm thickness. As the wire 51, nanofiber (for example, dtx44 of Toray Industries, Inc.) or the like can be used. Note that each rectangular opening in the frame 50 preferably has one side of few millimeters (approximately 2mm to 3mm) or more length, to prevent generation of a sidelobe by diffraction.

As stated above, the cap 30 can be easily formed of conventional resin material with low cost, while improving the consistency of the acoustic impedances between the cap 30 and the living organism. Accordingly, the receiver sensitivity of the ultrasonic transducer 31 is increased, a penetration of the obtained image is increased, and the performance of the ultrasonic diagnosing system 2 is improved.

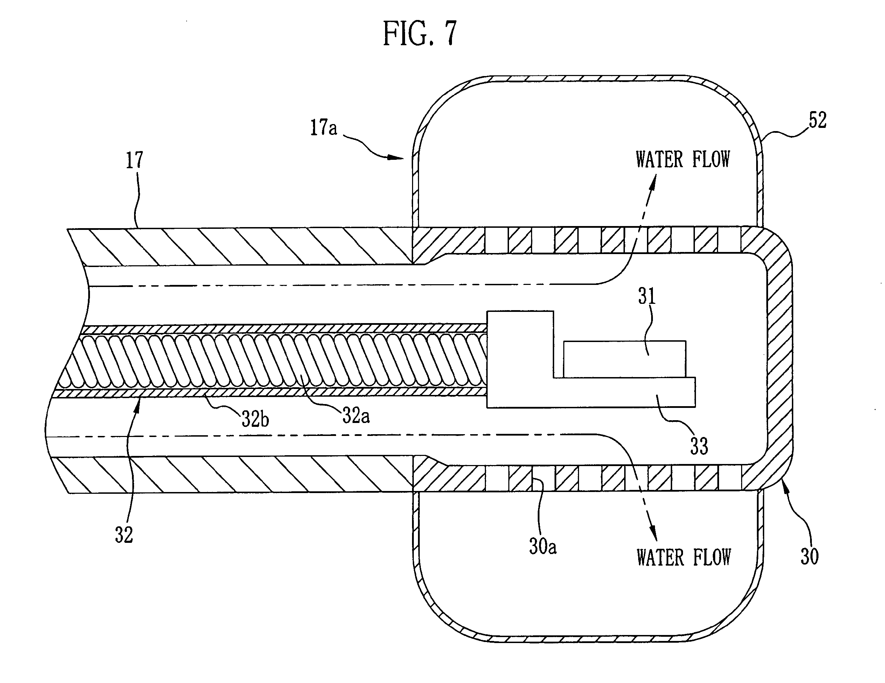

In an example shown in FIG.7, an elastic balloon 52 is attached to the cap 30 so as to cover the meshed area. Into the balloon 52, the water flows through the water inlet of the sheath 17, inside the sheath 17 and inside the cap 30. The balloon 52 expands according to an amount of entered water, to push the required portion in the living organism when the leading end 17a of the sheath 17 reaches the required portion. In this example, a conventional dedicated water supply line to the balloon 52 is not needed, because the sheath 17 and the cap 30 are worked as the water supply line.

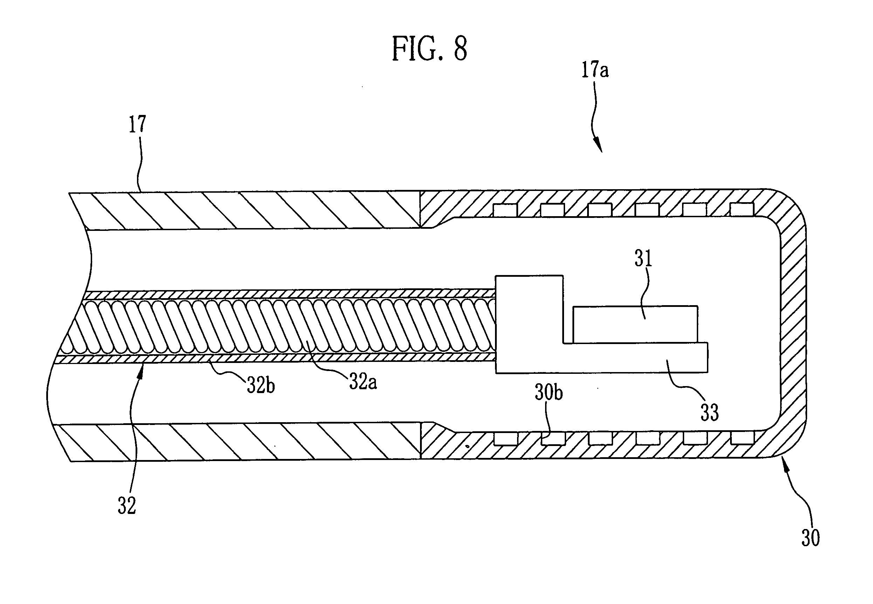

In the above embodiment, the hole 30a of the cap 30 has the rectangular shape. However, the shape of the hole 30a is not limited in the present invention, and any other shapes, for example a circular shape and a slit shape, can be applied. In addition, as shown in FIG.8, concaves 30b can be formed as alternative to the holes 30a. The concave 30b becomes the low density part in the high- and low-density area. Also in this case, when a density interval (pitch of the concaves 30b) is sufficiently smaller than the wavelength λ of the ultrasonic waves as describe above, the high- and low-density area is deemed uniform to the ultrasonic wave. In addition, the acoustic impedance of the cap 30 is reduced from when there is no concave 30b. In this embodiment, the water from the sheath 17 stays inside the cap 30.

In the above embodiments, the low material density parts (the holes 30a or the concaves 30b) are arranged at regular intervals, to give a constant acoustic impedance to whole of the high- and low-density area. However, according to the present invention the density interval is uneven in the high- and low-density area to give different acoustic impedances at different places in the high- and low-density area.

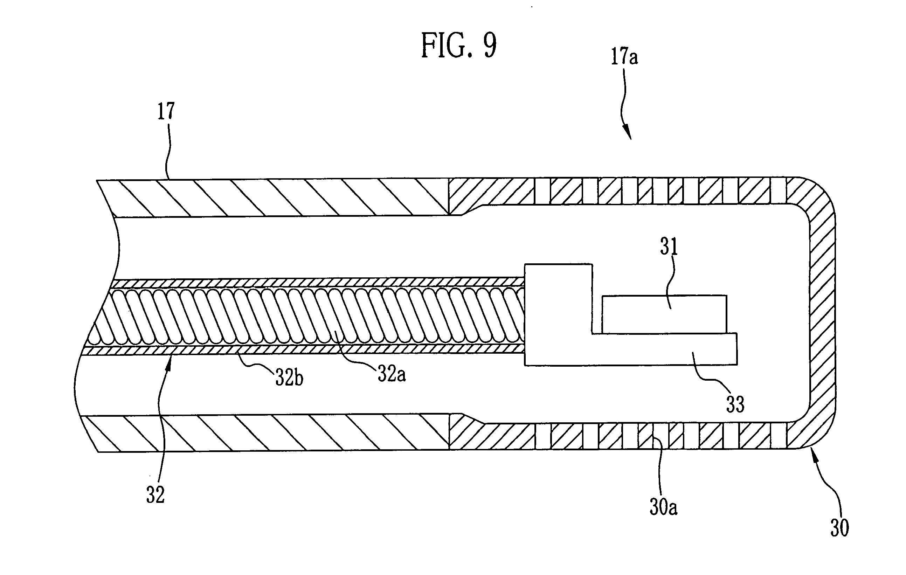

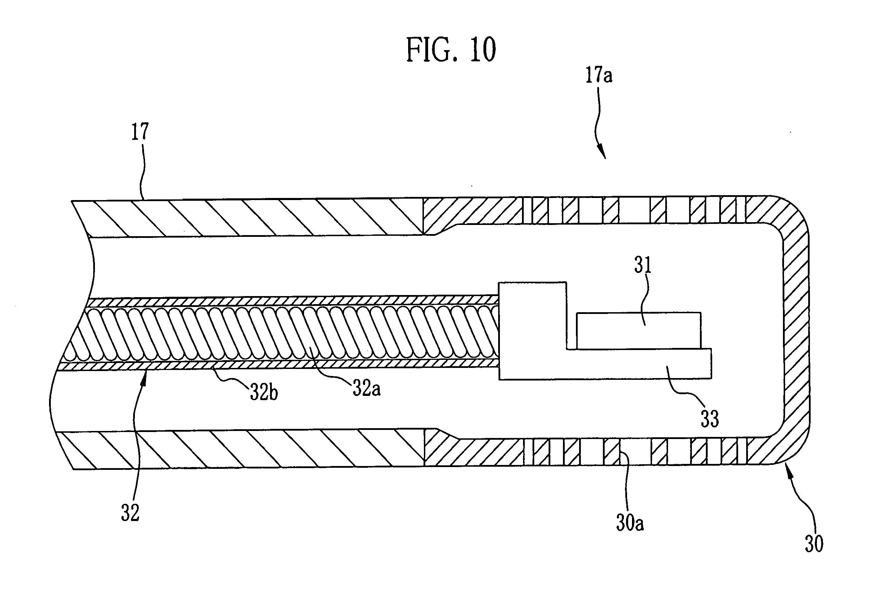

In FIG. 9, the interval between the low material density parts (holes 30a) gradually becomes larger from the center toward the ends in an axial direction of the cap 30. In FIG.10, the width of each hole 30a (interval between the high material density parts) gradually becomes smaller from the center toward the ends in the axial direction of the cap 30. In the both cases, in the cap 30, the acoustic impedance gradually becomes higher from the center, which corresponds to a center of the ultrasonic transducer 31, toward the ends in the axial direction. Accordingly, the cap 30 acts as an acoustic lens, which can focus the ultrasonic waves from the ultrasonic transducer 31 in the axial direction.

It may be also that the interval or width of the holes 30a is gradually changed in the circumference direction of the cap 30, to give the acoustic lens effect to the cap 30 in the circumference direction. In this case, the ultrasonic waves can be focused in the rotational direction of the ultrasonic transducer 31, which improves an azimuth resolution.

In the above embodiments, the high- and low-density area is formed only in a region of the cap 30 which the ultrasonic waves pass through. However, it may be that the high- and low-density area is formed in whole of the cap 30.

In the above embodiments, although water is supplied into the cap 30, the supplying liquid is not limited to the water. Another liquid, such as a saline or the like, can be used as an alternative to the water.

In the above embodiment, the ultrasonic probe 11 has the ultrasonic transducer which rotates around the flexible shaft 32a as the rotational axis. However, the present invention can be applied to an ultrasonic probe having the ultrasonic transducer which swings or slides to scan the living organism.

In the above embodiment, the ultrasonic transducer 31 sends and receives the ultrasonic. However, the present invention can be applied to an ultrasonic transducer which can only send or receive the ultrasonic.

Although the present invention has been fully described by way of the preferred embodiments thereof with reference to the accompanying drawings, various changes and modifications will be apparent to those having skill in this field. Therefore, unless these changes and modifications depart from the scope of the present invention, they should be construed as included therein.

## Claims

1. An ultrasonic probe comprising an ultrasonic transducer (31) for sending and/or receiving ultrasonic waves to and/or from a living organism and a cap (30) for covering said ultrasonic transducer (31) while transmitting said ultrasonic waves, said cap (30) including:
a high- and low-density area where low material density parts (30a, 30b) and high material density parts are alternately formed, for transmitting said ultrasonic waves, a density interval, i. e. the pitch (d) between said low material density parts or between said high material density parts, of said high- and low-density area being smaller than a wavelength of said ultrasonic waves **characterized in that** said density interval is changed in an area through which said ultrasonic waves pass, to give different acoustic impedances at different places in said high-and low-density area.

2. An ultrasonic probe as described in claim 1, wherein said low material density parts are openings (30a).

3. An ultrasonic probe as described in claim 2, comprising means (17) adapted to supply liquid into said cap (30) such that the liquid then flows outside said cap (30) through said openings (30a).

4. An ultrasonic probe as described in claim 2, further comprising:
a balloon (52) attached to said cap (30) for covering said openings (30a) such that a liquid can flow into said balloon (52) through inside said cap (30) and said openings (30a).

5. An ultrasonic probe as described in claim 2, wherein said high- and low-density area is formed by winding a wire (51) around a frame (50).

6. An ultrasonic probe as described in claim 1, wherein said low material density parts are concaves (30b).

7. An ultrasonic probe as described in claim 1, wherein said cap (30) has a cylindrical shape and wherein said density interval is gradually changed in an axial direction and/or a circumference direction of said cap (30).

8. An ultrasonic probe as described in claim 7, wherein said density interval is gradually changed from the center toward the ends in said axial direction of said cap (30) such that said acoustic impedance gradually becomes higher from said center toward said ends, so as to give an acoustic lens effect to said cap (30) in said axial direction.

## Patentansprüche

1. Ultraschallsonde, umfassend einen Ultraschallwandler (31) zum Senden und/oder Empfangen von Ultraschallwellen zu und/oder von einem lebenden Organismus, und eine Kappe (30) zum Abdecken des Ultraschallwandlers (31) während des Sendens der Ultraschallwellen, wobei die Kappe (30) aufweist:
einen Bereich hoher und geringer Dichte, in welchem Teile geringer Materialdichte (30a, 30b) und Teile hoher Materialdichte abwechselt ausgebildet sind zum Senden der Ultraschallwellen, wobei ein Dichteintervall, d. h. der Mittenabstand (d) zwischen den Teilen geringer Materialdichte oder zwischen den Teilen hoher Materialdichte des Bereichs hoher und geringer Dichte kleiner ist als eine Wellenlänge der Ultraschallwellen, **dadurch gekennzeichnet, dass** das Dichteintervall in einem Bereich, durch den die Ultraschallwellen hindurchgehen, geändert ist, um an verschiedenen Stellen innerhalb des Bereichs hoher und geringer Dichte unterschiedliche akustische Impedanzen zu erhalten.

2. Sonde nach Anspruch 1, bei der die Teile geringer Materialdichte Öffnungen (30a) sind.

3. Sonde nach Anspruch 2, umfassend eine Einrichtung (17), die dazu ausgebildet ist, Flüssigkeit in die Kappe (30) zu liefern, demzufolge die Flüssigkeit dann durch die Öffnungen (30a) aus der Kappe (30) ausströmt.

4. Sonde nach Anspruch 2, weiterhin umfassend:
einen an der Kappe (30) zum Bedecken der Öffnung (30a) angebrachten Ballon (52), so dass eine Flüssigkeit durch das Innere der Kappe (30) und die Öffnungen (30a) in den Ballon (52) einströmen kann.

5. Sonde nach Anspruch 2, bei der der Bereich hoher und geringer Dichte durch Wickeln eines Drahtes (51) um ein Gestell (50) gebildet ist.

6. Sonde nach Anspruch 1, bei der die Teile geringer Materialdichte Ausnehmungen (30b) sind.

7. Sonde nach Anspruch 1, bei der die Kappe (30) zylindrische Form hat, wobei das Dichteintervall in axialer Richtung und/oder in Umfangsrichtung der Kappe (30) allmählich geändert ist.

8. Sonde nach Anspruch 7, bei der das Dichteintervall von der Mitte in Richtung der Enden in axialer Richtung der Kappe (30) allmählich derart geändert ist, dass die akustische Impedanz von der Mitte zu den Enden hin allmählich größer wird, um der Kappe (30) in axialer Richtung den Effekt einer akustischen Linse zu verleihen.

## Revendications

1. Sonde ultrasonore comprenant un transducteur ultrasonore (31) pour envoyer et / ou recevoir des ondes ultrasonores vers et / ou d'un organisme vivant et un capuchon (30) pour recouvrir ledit transducteur ultrasonore (31) tout en transmettant lesdites ondes ultrasonores, ledit capuchon (30) comprenant :
des zones de forte et de faible densité où les parties de faible densité de matériau (30a, 30b) et les parties de forte densité de matériau sont formées alternativement pour transmettre lesdites ondes ultrasonores, un intervalle de densité, c'est-à-dire, le pas (d) entre lesdites parties de faible densité de matériau ou entre lesdites parties de forte densité de matériau, desdites zones de forte et de faible densité étant inférieur à une longueur d'onde desdites ondes ultrasonores, **caractérisée en ce que** ledit intervalle de densité est modifié dans une zone à travers laquelle lesdites ondes ultrasonores passent, pour donner différentes impédances acoustiques à différents emplacements dans lesdites zones de forte et de faible densité.

2. Sonde ultrasonore selon la revendication 1, dans laquelle lesdites parties de faible densité de matériau sont des ouvertures (30a).

3. Sonde ultrasonore selon la revendication 2, comprenant des moyens (17) adaptés pour délivrer un liquide dans ledit capuchon (30) de sorte que le liquide circule ensuite à l'extérieur dudit capuchon (30) à travers lesdites ouvertures (30a).

4. Sonde ultrasonore selon la revendication 2, comprenant en outre :
un ballonnet (52) fixé audit capuchon (30) pour recouvrir lesdites ouvertures (30a) de sorte qu'un liquide puisse circuler dans ledit ballonnet (52) à travers l'intérieur dudit capuchon (30) et desdites ouvertures (30a).

5. Sonde ultrasonore selon la revendication 2, dans laquelle lesdites zones de forte et de faible densité sont formées en enroulant un fil (51) autour d'un cadre (50).

6. Sonde ultrasonore selon la revendication 1, dans laquelle lesdites parties de faible densité de matériau sont concaves (30b).

7. Sonde ultrasonore selon la revendication 1, dans laquelle ledit capuchon (30) a une forme cylindrique, et dans laquelle ledit intervalle de densité est modifié graduellement dans une direction axiale et / ou une direction circonférentielle dudit capuchon (30).

8. Sonde ultrasonore selon la revendication 7, dans laquelle ledit intervalle de densité est modifié graduellement du centre vers les extrémités dans ladite direction axiale dudit capuchon (30) de sorte que ladite impédance acoustique devienne graduellement plus élevée dudit centre vers lesdites extrémités, de manière à communiquer un effet de lentille acoustique audit capuchon (30) dans ladite direction axiale.
